# EUROPEAN PATENT SPECIFICATION

(11) **EP 2 358 910 B1**
(45) Date of publication and mention of the grant of the patent: **22.11.2017**
(21) Application number: 09827253.7
(22) Date of filing: 19.11.2009
(51) Int. Cl.: C12Q 1/68, C12Q 1/70

(54) **NUCLEOTIDE SEQUENCES, METHODS AND KITS FOR DETECTING HPV**
NUKLEOTIDSEQUENZEN, VERFAHREN UND KITS FÜR DEN NACHWEIS VON HPV
SÉQUENCES NUCLÉOTIDIQUES, PROCÉDÉS ET KITS POUR DÉTECTER LE VPH

(30) Priority: 19.11.2008 HK 08112649
(43) Date of publication of application: 24.08.2011
(73) Proprietor: DiagCor Life Science Limited, Kowloon Bay HongKong (CN)
(72) Inventor: TAM, Joseph Wing On, Hong Kong n.a. (CN); CHOW, Kwok Fai Joseph, Hong Kong n.a. (CN); CHAN, Rhys Cheuk Yu, Hong Kong n.a. (CN)
(74) Representative: inCompass IP Europe Limited
(86) International application number: PCT/IB2009/055158
(87) International publication number: WO 2010/058357

(56) References cited:
- WO-A1-95/22626
- WO-A1-2007/100198
- WO-A2-99/14377
- CN-A- 1 814 796
- CN-A- 101 177 701
- US-A1- 2005 175 989
- US-A1- 2005 175 989
- US-A1- 2006 121 516
- KORNEGAY J R ET AL: "Nonisotopic detection of human papillomavirus DNA in clinical specimens using a consensus PCR and a generic probe mix in an enzyme-linked immunosorbent assay format", JOURNAL OF CLINICAL MICROBIOLOGY, AMERICAN SOCIETY FOR MICROBIOLOGY, WASHINGTON, DC, US, vol. 39, no. 10, 1 October 2001 (2001-10-01), pages 3530-3536, XP002342718, ISSN: 0095-1137, DOI: 10.1128/JCM.39.10.3530-3536.2001
- MEYER T ET AL: "Strategy for typing human papillomaviruses by RFLP analysis of PCR products and subsequent hybridization with a generic probe", BIOTECHNIQUES, INFORMA HEALTHCARE, US, vol. 19, no. 4, 1 January 1995 (1995-01-01), pages 632-639, XP009122660, ISSN: 0736-6205
- DE VILLIERS E-M ET AL: "Classification of papillomaviruses", VIROLOGY, ACADEMIC PRESS,ORLANDO, US, vol. 324, no. 1, 20 June 2004 (2004-06-20) , pages 17-27, XP004512668, ISSN: 0042-6822, DOI: 10.1016/J.VIROL.2004.03.033
- FRANC,OIS COUTLE'E ET AL.: 'Enhanced Detection and Typing of Human Papillomavirus (HPV) DNA in Anogenital Samples with PGMY Primers and the Linear Array HPV Genotyping Test' JOURNAL OF CLINICAL MICROBIOLOGY 30 June 2006, pages 1998 - 2006, XP008148664
- MUÑOZ NUBIA ET AL: "Epidemiologic Classification of Human Papillomavirus Types Associated with Cervical Cancer", NEW ENGLAND JOURNAL OF MEDICINE, THE, MASSACHUSETTS MEDICAL SOCIETY, WALTHAM, MA, US, vol. 348, no. 6, 6 February 2003 (2003-02-06), pages 518-527, XP008142603, ISSN: 0028-4793, DOI: 10.1056/NEJMOA021641
- Weimin Qu ET AL: "PCR Detection of Human Papillomavirus: Comparison between MY09/MY11 and GP5ϩ/GP6ϩ Primer Systems", Copyright, 1 January 1997 (1997-01-01), pages 1304-1310, XP055089536, Retrieved from the Internet: URL:http://www.ncbi.nlm.nih.gov/pmc/articl es/PMC229739/pdf/351304.pdf [retrieved on 2013-11-21]

### SEQUENCE LISTING

<110> DiagCor Bioscience Incorporation Ltd.
   TAM, Joseph
   CHOW, Kwok Fai Joseph
   CHAN, Rhys Cheuk Yu
<120> Sequences, Methods, and Systems for Detecting HPV
<130> 22050
<150> HK08112649.2
   <151> 2008-11-19
<160> 32
<170> PatentIn version 3.5
<210> 1
   <211> 36
   <212> DNA
   <213> Artificial Sequence
<220>
   <223> HPV Probe
<220>
   <221> misc_feature
   <222> (6) .. (6)
   <223> n is a, c, g, t or u
<400> 1
   catgbnggag gagtttgatt tacaatttat gtttca 36
<210> 2
   <211> 29
   <212> DNA
   <213> Artificial Sequence
<220>
   <223> HPV Probe
<400> 2
   gaagagtatg atttgcaatt tatatttca 29
<210> 3
   <211> 23
   <212> DNA
   <213> Artificial Sequence
<220>
   <223> HPV Probe
<400> 3
   tttgttactg tggtagatac tac 23
<210> 4
   <211> 25
   <212> DNA
   <213> Artificial Sequence
<220>
   <223> HPV Probe
<400> 4
   gaaaaataaa ctgtaaatca tattc 25
<210> 5
   <211> 20
   <212> DNA
   <213> Artificial Sequence
<220>
   <223> HPV Probe
<220>
   <221> misc_feature
   <222> (3) .. (3)
   <223> n is a, c, g, t or u
<220>
   <221> misc_feature
   <222> (9) .. (9)
   <223> n is a, c, g, t or u
<400> 5
   gcncagggnc ataacaatgg 20
<210> 6
   <211> 20
   <212> DNA
   <213> Artificial Sequence
<220>
   <223> HPV probe
<220>
   <221> misc_feature
   <222> (3) .. (3)
   <223> n is a, c, g, t or u
<220>
   <221> misc_feature
   <222> (9) .. (9)
   <223> n is a, c, g, t or u
<400> 6
   gcncagggnc acaataatgg 20
<210> 7
   <211> 25
   <212> DNA
   <213> Artificial Sequence
<220>
   <223> HPV Probe
<220>
   <221> misc_feature
   <222> (20) .. (21)
   <223> n is a, c, g, t or u
<400> 7
   aatgtaaatc atattcctcn ncatg 25
<210> 8
   <211> 25
   <212> DNA
   <213> Artificial Sequence
<220>
   <223> HPV Probe
<400> 8
   ctgttgttga tactacacgc agtac 25
<210> 9
   <211> 25
   <212> DNA
   <213> Artificial Sequence
<220>
   <223> HPV Probe
<400> 9
   ctgtggtwga taccacwcgc agtac 25
<210> 10
   <211> 23
   <212> DNA
   <213> Artificial Sequence
<220>
   <223> HPV Probe
<400> 10
   gaggaatatg attgacagtt taa 23
<210> 11
   <211> 25
   <212> DNA
   <213> Artificial Sequence
<220>
   <223> HPV Probe
<400> 11
   aaataaactg taaatcatat tcctc 25
<210> 12
   <211> 26
   <212> DNA
   <213> Artificial Sequence
<220>
   <223> HPV Probe
<220>
   <221> misc_feature
   <222> (2) .. (2)
   <223> n is a, c, g, t or u
<400> 12
   bnggaggaat atgattgaca gtttat 26
<210> 13
   <211> 28
   <212> DNA
   <213> Artificial Sequence
<220>
   <223> HPV Probe
<220>
   <221> misc_feature
   <222> (2) .. (2)
   <223> n is a, c, g, t or u
<400> 13
   bnggaggaat atgatttaca gtttattt 28
<210> 14
   <211> 35
   <212> DNA
   <213> Artificial Sequence
<220>
   <223> HPV Probe
<220>
   <221> misc_feature
   <222> (6) .. (6)
   <223> n is a, c, g, t or u
<400> 14
   catgbngarg aatatgattt gcaatttatw tttca 35
<210> 15
   <211> 29
   <212> DNA
   <213> Artificial Sequence
<220>
   <223> HPV Probe
<400> 15
   gargaatwtg atttrcagtt tatatttca 29
<210> 16
   <211> 18
   <212> DNA
   <213> Artificial Sequence
<220>
   <223> HPV Probe
<400> 16
   aayaatggta tytgytgg 18
<210> 17
   <211> 18
   <212> DNA
   <213> Artificial Sequence
<220>
   <223> HPV Probe
<400> 17
   aataatggca tytgttgg 18
<210> 18
   <211> 18
   <212> DNA
   <213> Artificial Sequence
<220>
   <223> HPV Probe
<400> 18
   aataatggta tytgctgg 18
<210> 19
   <211> 18
   <212> DNA
   <213> Artificial Sequence
<220>
   <223> HPV Probe
<400> 19
   aacaatggta tytgctgg 18
<210> 20
   <211> 18
   <212> DNA
   <213> Artificial Sequence
<220>
   <223> HPV Probe
<400> 20
   aacaatggta tytgttgg 18
<210> 21
   <211> 20
   <212> DNA
   <213> Artificial Sequence
<220>
   <223> HPV Probe
<220>
   <221> misc_feature
   <222> (3) .. (3)
   <223> n is a, c, g, t or u
<220>
   <221> misc_feature
   <222> (9) .. (9)
   <223> n is a, c, g, t or u
<400> 21
   gcncagggnc ataacaatgg 20
<210> 22
   <211> 23
   <212> DNA
   <213> Artificial Sequence
<220>
   <223> HPV Probe
<220>
   <221> misc_feature
   <222> (12) .. (12)
   <223> n is a, c, g, t or u
<220>
   <221> misc_feature
   <222> (15) .. (15)
   <223> n is a, c, g, t or u
<400> 22
   tttgttactg tngtngatac cac 23
<210> 23
   <211> 23
   <212> DNA
   <213> Artificial Sequence
<220>
   <223> HPV Probe
<400> 23
   gaggaatatg attgacagtt taa 23
<210> 24
   <211> 25
   <212> DNA
   <213> Artificial Sequence
<220>
   <223> HPV Probe
<400> 24
   gaaaaataaa ctgtaaatca tattc 25
<210> 25
   <211> 20
   <212> DNA
   <213> Artificial Sequence
<220>
   <223> Primer for amplification of HPV
<400> 25
   gcmcagggwc ataayaatgg 20
<210> 26
   <211> 20
   <212> DNA
   <213> Artificial Sequence
<220>
   <223> Primer for amplification of HPV
<400> 26
   cgtccmarrg gawactgatc 20
<210> 27
   <211> 20
   <212> DNA
   <213> Artificial Sequence
<220>
   <223> Primer for amplification of HPV
<400> 27
   gcgacccaat gcaaattggt 20
<210> 28
   <211> 20
   <212> DNA
   <213> Artificial Sequence
<220>
   <223> Primer for amplification of HPV
<400> 28
   ggtccmarrg gawactgatc 20
<210> 29
   <211> 21
   <212> DNA
   <213> Artificial Sequence
<220>
   <223> Globin primer
<400> 29
   ccttgatacc aacctgccca g 21
<210> 30
   <211> 21
   <212> DNA
   <213> Artificial Sequence
<220>
   <223> Globin primer
<400> 30
   gtgcacctga ctcctgagga g 21
<210> 31
   <211> 26
   <212> DNA
   <213> Artificial Sequence
<220>
   <223> Globin probe
<400> 31
   aaggtgaacg tggatgaagt tggtgg 26
<210> 32
   <211> 20
   <212> DNA
   <213> Artificial Sequence
<220>
   <223> Globin primer (new)
<400> 32
   gaagagccaa ggacaggtac 20

## Description

### Field of the Invention

This invention relates to nucleotide sequences, methods and kits for detecting human papillomavirus (HPV), particularly those for detecting multiple subtypes of HPV with a relatively small number of nucleotide sequences as probes.

### Background of the Invention

Cervical cancer is one of the most common cancers in woman. The worldwide death rate was estimated to be 273,500 in 2002, and increased to 320,000 in 2004. It was estimated that 83% of these cases are in developing countries, accounting for 15 - 24% in cancers in woman, which are much higher than the 3 to 7% in developed countries. Cervical cancer was found to be closely related to human papillomavirus (HPV) infection, which causes a precancerous stage, cervical intraepithelial neoplasia (CIN), during the cancer development. Therefore, HPV detection plays an important role in preventing and treating cervical cancer.

The methods for detection of HPV in human using nucleotide sequences can be classified into two groups. Those of the first group differentiate the subtypes of HPV while detecting the presence of HPV in human samples, and examples includes Roche® AMPLICOR™ HPV Test and LINEAR ARRAY™ HPV Genotyping Test; Innogentics® INNO-LiPA™ HPV Genotyping Extra; CN200410037617.7 of Decipher Bioscience (Shenzhen) LTD; and CN200610061044.0 of Longyang Bioscience (Shenzhen) LTD. This group of methods generally require the presence of one nucleotide probe specific for each of the subtypes of HPV to be detected and these probes must be in separate locations (i.e. in individual spot or line as used in the given examples above on the array format or in different wells of the 96 wells ELISA format (Boehringer, Mannheim) and by some other methods alternative to the arrays, and therefore, is more complicated to develop and generally more expensive. Most importantly, the fact that there are more than 200 HPV subtypes have been isolated and more new ones are expected and consequently all these existing genotyping test kits are not capable of detecting the HPV viruses other than those 20-37 types specified by each HPV test kits made available currently preventing definitive detection on the Presence/absence of the HPV virus because specific probes are needed for each and every subtype to be tested as claimed. Although it is possible to add more types in the genotyping format but the validation process (especially for rare types) required by respective regulatory agency makes it difficult if not impossible because rare types difficult to obtain from clinical samples. Hence the genotyping approach for definitive typing to cover all HPV subtypes is not only difficult to develop and expensive but also less feasible.

Those of the second group only detect the presence of HPV without differentiating the subtypes of HPV, in which Digene Hybrid Capture® II (HCII) is the only currently available commercial product known to the inventors. The HCII is further subdivided into high risk and low risk types, which detect HPV DNA directly using liquid hybridization in clinical samples without amplification of the target viral DNA and instead it uses the chemiluminescence signal amplification technology. Hence in general, the signal to noise ratio is low at relatively low target concentration and therefore the detection sensitivity is much lower that that of the PCR based test. In addition, other than the use of RNA probe may pose low stability of the kit and the possibility of contamination (the HCII is dependent on the cellular content of samples, rendering occasionally false positive and false negative results, i.e. nearby its detection cut off concentration the false positive/negative rates are reportedly higher), the HCII is also expensive. Further, for HCII, specific RNA probes are used to capture the target subtype DNA to be tested, and therefore HCII also faces development limitations similar to those of the first group above (the genotyping assay), except that it is done in the liquid mixture without genotyping. Additionally, the HCII is able to detect only 18 subtypes of HPV (13 in HCII high risk type, and 5 in HCII low risk type), which are far less than the most commonly seen 38 subtypes of HPV in human. Some of the 150 plus rare subtypes of HPV are already confirmed high risk for cancer induction, and hence the ability of detecting these subtypes is also important to the diagnosis and prevention of cervical cancer.

WO2007/100198 discloses kits and methods for detecting HPV with an oligonucleotide bead array; WO99/14377 discloses detection of HPV by PCR and type-specific reverse hybridization; US2006/121516 discloses probes useful in detection of HPV; Kornegay et al (2001). J. Clin. Microbiol.vol-39, no. 10, p3530 discloses use of consensus PCR and a probe mix for HPV detection in an ELISA format; and Weimin Qu et al: "PCR Detection of Human Papillomavirus: Comparison between MY09/MY11 and GP5+/GP6+ Primer Systems", Journal of Clinical Microbiology, 1 June 1997, p1304-1310 discloses the use of specific PCT primer sets for the detection of HPV DNA in cervicovaginal lavage.

Under these circumstances, there are needs for exploring and developing a simple and/or cost-effective method for detection of HPV infection of various subtypes.

### Objects of the Invention

Therefore, it is an object of this invention to resolve at least one or more of the problems as set forth in the prior art. Particularly, it is an object of the current invention to provide nucleotide sequences and related methods and kits for detecting commonly seen subtypes of HPV in a sample at reduced costs. As a minimum, it is an object of this invention to provide the public with a useful choice.

### Summary of the Invention

Accordingly, this invention provides an isolated nucleic acid molecule selected from the group consisting of a sequence selected from the group consisting of SEQ ID NOS: 1, 2 and 4 as shown in table 1, wherein a + sign preceding a base of SEQ ID NOS: 1, 2 and 4 means that the base is an LNA modified base.

It is another aspect of this invention to provide an HPV detection kit, which includes:
a) at least one HPV probe for detecting HPV, each HPV probe being an isolated nucleic acid molecule selected from the group consisting of a sequence selected from the group consisting of SEQ ID NOS: 1, 2 and 4 as shown in table 1; wherein a + sign preceding a base of SEQ ID NOS: 1, 2 and 4 means that the base is an LNA modified base;
b) primers for amplifying HPV in sample DNA by polymerase chain reaction; and
c) labels for labeling DNA that is amplified and hybridized to said DNA chip.

It is yet another aspect of the current invention to provide a DNA chip for detecting at least one HPV probe, each HPV probe being an isolated nucleic acid molecule selected from the group consisting of a sequence selected from the group consisting of SEQ ID NOS: 1, 2 and 4 as shown in table 1; wherein a + sign preceding a base of SEQ ID NOS: 1, 2 and 4 means that the base is an LNA modified base.

It is a further aspect of the current invention to provide a method of detecting presence of HPV in a sample, including the steps of: amplifying DNA of HPV present in the sample by means of a polymerase chain reaction; detecting HPV with at least one HPV probe, each HPV probe being an isolated nucleic acid molecule selected from the group consisting of a sequence selected from the group consisting of SEQ ID NOS: 1, 2 and 4 as shown in table 1; wherein a + sign preceding a base of SEQ ID NOS: 1, 2 and 4 means that the base is an LNA modified base.

The current invention also provides an HPV detection kit, a DNA chip or a method including a combination of four HPV probes to detect HPV, said four HPV probes being sequences consisting of SEQ ID NOS. 1-3 and 5, as shown in table 1; sequences consisting of SEQ ID NOS. 1-3 and 6, as shown in table 1; sequences consisting of SEQ IS NOS. 1, 2, 4 and 5, as shown in table 1; or sequences consisting of SEQ ID NOS : 1, 2, 4 and 7, as shown in table 1; or a combination of four HPV probes to detect HPV, said four HPV probes consisting of sequences consisting of SEQ ID NOS: 1-4, as shown in table 1, or sequences consisting of SEQ ID NOS: 2, 6, 22 and 24, as shown in table 1; or a combination of three HPV probes to detect HPV, the combination of the said three HPV probes consists of SEQ ID NOS: 2, 3 and 5, as shown in table 1; or a combination of two HPV probes to detect HPV, the combination of said two HPV probes consisting of SEQ ID NO: 1 and one of SEQ ID NOS: 2, 3, 4, 16 and 24, as shown in table 1.

The current invention also provides an HPV detection kit including: at least two HPV probes for detecting HPV, one of said at least two HPV probes being an isolated nucleic acid molecule consisting of SEQ ID NO: 1, as shown in table 1, the other one of said at least two HPV probes being an isolated nucleic acid molecule consisting of SEQ ID NOS: 2, 3, 4, 16, or 24, as shown in table 1, wherein a + sign preceding a base of SEQ ID NOS: 1, 2, 3, 4, 16 and 24 means that the base is an LNA modified base; primers for amplifying HPV in sample DNA by polymerase chain reaction; and labels for labeling DNA that is amplified and hybridized to a DNA chip.

The current invention also provides a method of detecting presence of HPV, including the steps of: amplifying DNA of HPV present in the sample by means of a polymerase chain reaction; detecting HPV with at least two HPV probes, one of the said HPV probes being an isolated nucleic acid molecule consisting of SEQ ID NO:1 as shown in table 1, the other one of said at least two HPV probes being an isolated nucleic acid molecule consisting of SEQ ID NOS: 2, 3, 4, 16, or 24, as shown in table 1 when said one of said at least two HPV probes is SEQ ID NO: 1, as shown in table 1; wherein a + sign preceding a base of SEQ ID NOS: 1, 2, 3, 4, 16 and 24 means that the base is an LNA modified base.

In any of the above various aspects of the current invention, preferably the isolated nucleic acid molecule is DNA.

### Brief description of the drawing

Preferred embodiments of the present invention will now be explained by way of example and with reference to the accompanying drawing in which:

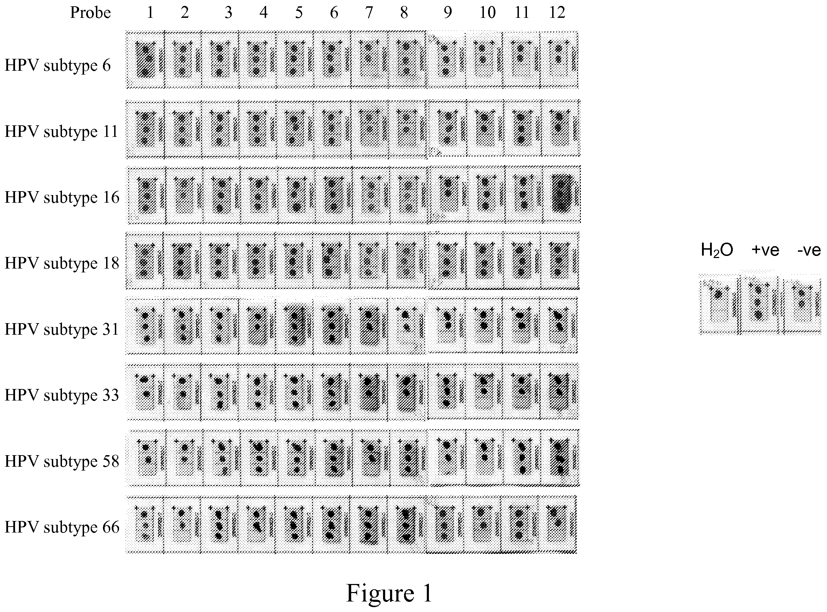

**Figure 1** shows the images of the detection of various subtypes of HPV by exemplary nucleotide sequences of the current invention.

### Detailed Description of the Preferred Embodiments

This invention is now described by way of example with reference to the figure in the following paragraphs. Objects, features, and aspects of the present invention are disclosed in or are apparent from the following description. It is to be understood by one of ordinary skilled in the art that the present discussion is a description of exemplary embodiments only, and is not intended as limiting the broader aspects of the present invention, which broader aspects are embodied in the exemplary constructions.

The present inventors have prepared detection probes for various subtypes of HPV having nucleotide sequences complementary to the DNA of HPV such that relatively few probes can detect most of the commonly seen subtypes of HPV. In a particular embodiment, at least 36 most commonly seen subtypes of HPV can be detected by various combinations of 2 to 4 HPV probes of the current invention. The probes of the present disclosure comprise nucleotide sequences set forth in SEQ ID NOS: 1 - 24 **(Table 1).**

**Table 1 - sequences of nucleotides of the current invention used as probes for detecting multiple subtypes of HPV (B=C/G/T; M=A/C; W=A/T; Y=T+C; N=A/C/G/T. LNA modified base is marked by (+) in front of the base)**

| **Name** | **SEQ ID NO** | **Sequence** | **5' Modification** | **Length** |
|---|---|---|---|---|
| HPV Probe 1 | 1 | CATGB NGGAG G+AGTT TGATT TACAA T+TTAT GT+TTC A | Amine | 36 |
| HPV Probe 2 | 2 | GAAGA GTA+TG ATTTG CAATT TATAT+ TTCA | Amine | 29 |
| HPV Probe 3 | 3 | TTTG+T TACTG TGGTA GAT+AC TAC | Amine | 23 |
| HPV Probe 4 | 4 | GA+AAAATA+AACTGTAAATCATAT+TC | Amine | 25 |
| HPV Probe 5 | 5 | GCNCA GGGNC ATAAC AATGG | Amine | 20 |
| HPV Probe 6 | 6 | GCNCA GGGNC ACAAT AATGG | Amine | 20 |
| HPV Probe 7 | 7 | AATGT AAATC ATATT CCTCN NCATG | Amine | 25 |
| HPV Probe 8 | 8 | CTGTT GTTGA TACTA CACGC AGTAC | Amine | 25 |
| HPV Probe 9 | 9 | CTGTG GTWGA TACCA CWCGC AGTAC | Amine | 25 |
| HPV Probe 10 | 10 | GAGGA ATATG ATTGA CAGTT TAA | Amine | 23 |
| HPV Probe 11 | 11 | AAATA AACTG TAAAT CATAT TCCTC | Amine | 25 |
| HPV Probe 12 | 12 | BNGGA GGAAT ATGAT TGACA GTTTA T | Amine | 26 |
| HPV Probe 13 | 13 | BNGGA GGAAT ATGAT TTACA GTTTA TTT | Amine | 28 |
| HPV Probe 14 | 14 | CATGB NGARG AATAT GATTT GCAAT TTATW TTTCA | Amine | 35 |
| HPV Probe 15 | 15 | GARGA ATWTG ATTTR CAGTT TATAT TTCA | Amine | 29 |
| HPV Probe 16 | 16 | AAY+AA TGGTA TY+TGY+ TGG | Amine | 18 |
| HPV Probe 17 | 17 | AAT+AATGGCATY+TGT+TGG | Amine | 18 |
| HPV Probe 18 | 18 | AAT+AATGGTATY+TGC+TGG | Amine | 18 |
| HPV Probe 19 | 19 | AAC+AATGGTATY+TGC+TGG | Amine | 18 |
| HPV Probe 20 | 20 | AAC+AATGGTATY+TGT+TGG | Amine | 18 |
| HPV Probe 21 | 21 | GCNCA GGGNC ATAAC AATGG | Amine | 20 |
| HPV Probe 22 | 22 | TTTGT TACTG TNGTN GATAC CAC | Amine | 23 |
| HPV Probe 23 | 23 | GAGGA ATATG ATTGA CAGTT TAA | Amine | 23 |
| HPV Probe 24 | 24 | GA+AAA ATAAA CTGTA AATCA TATTC | Amine | 25 |

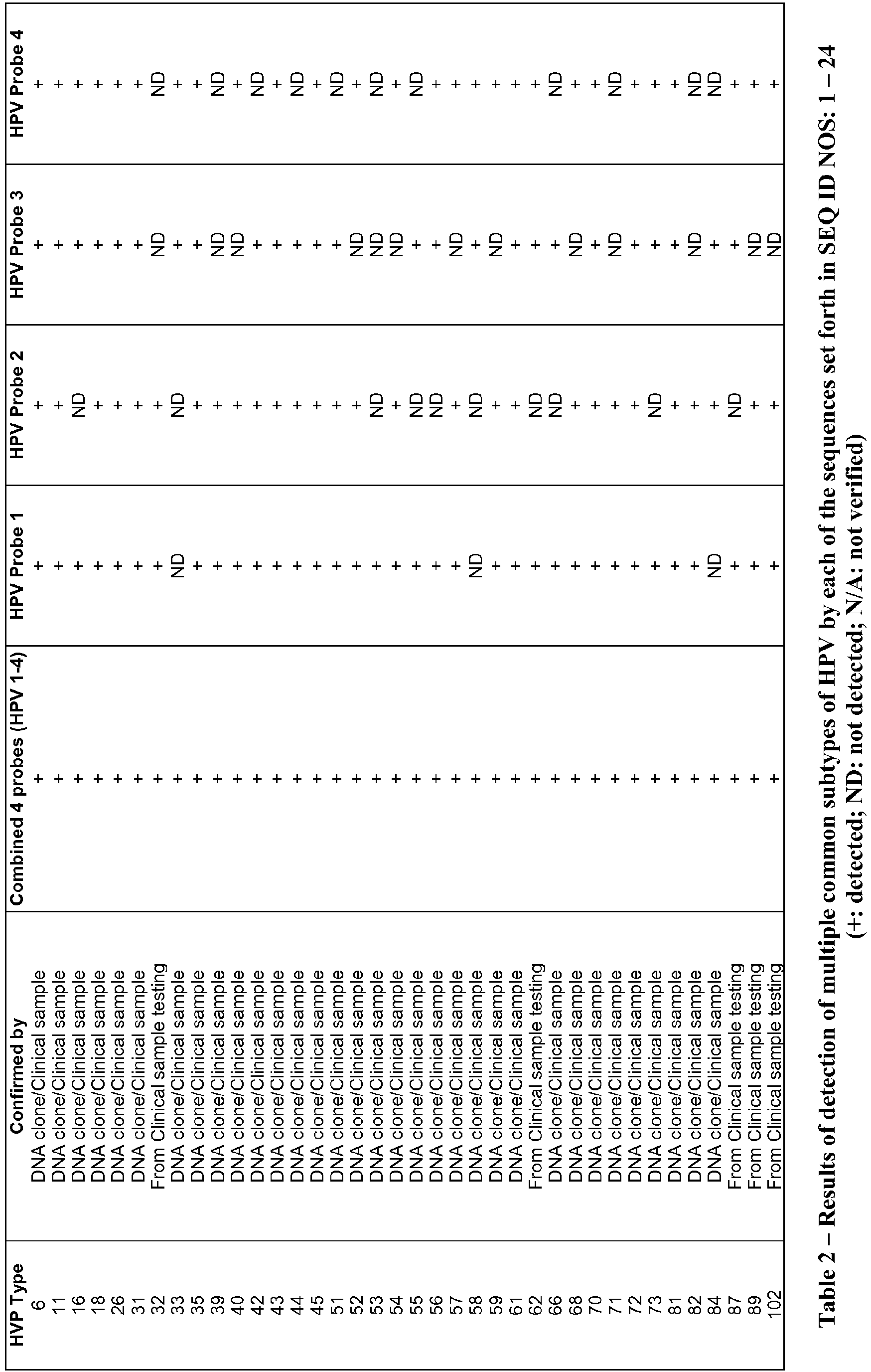

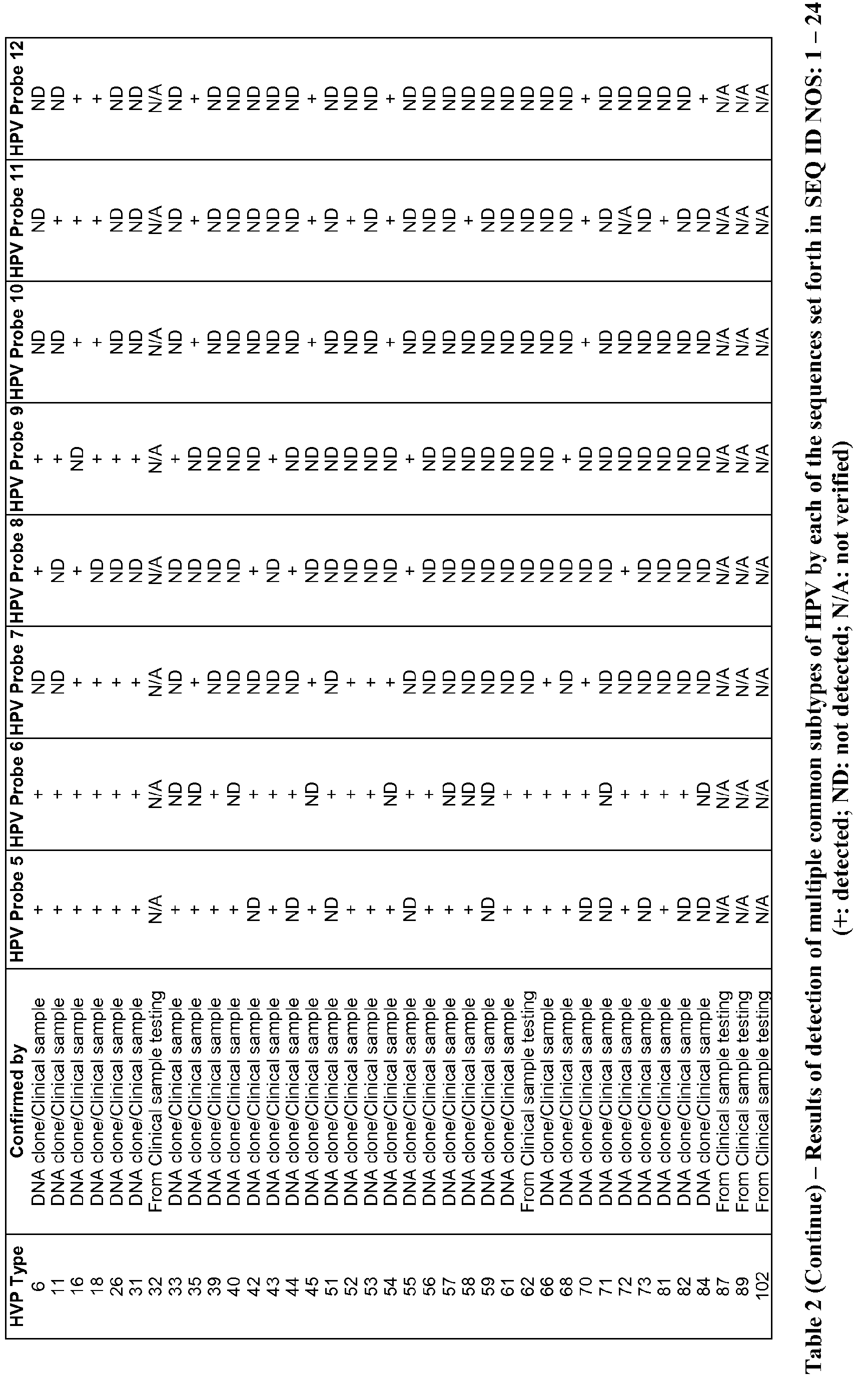

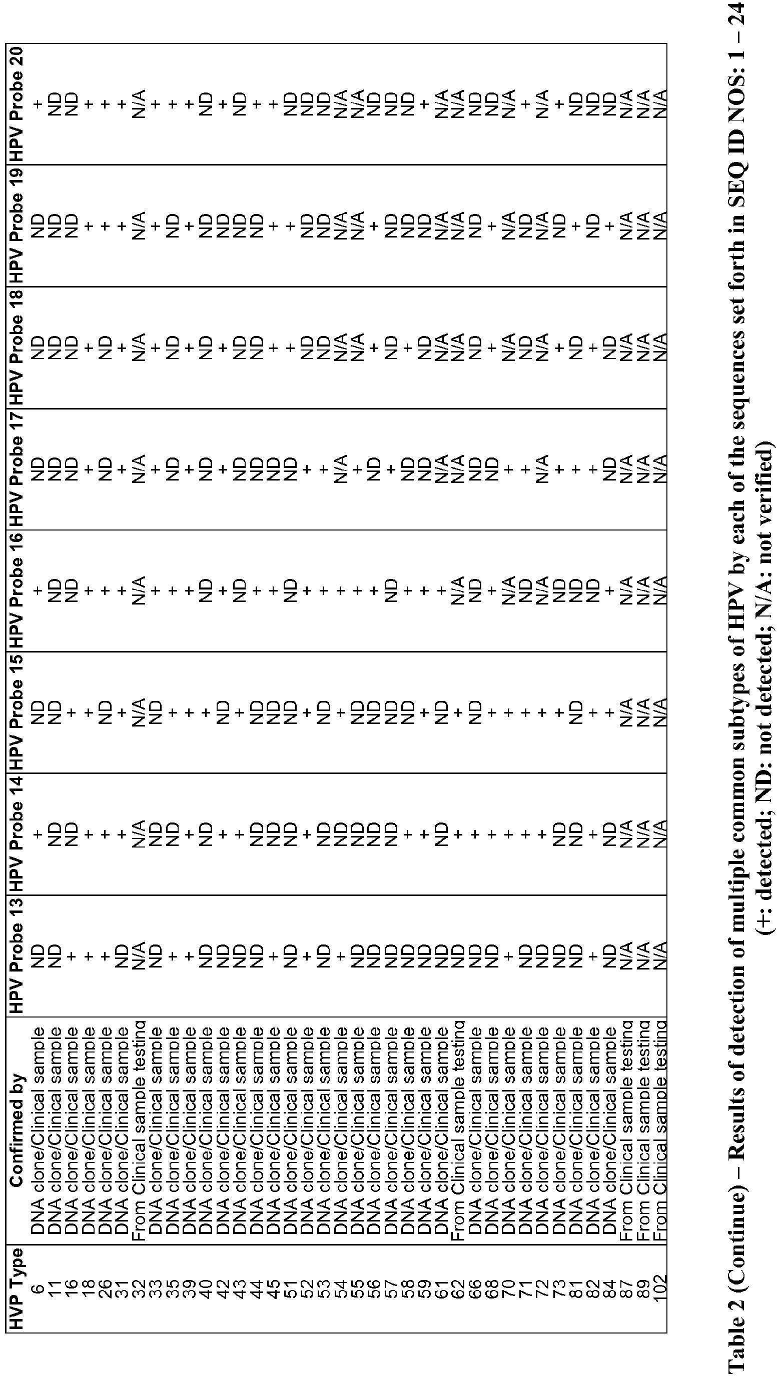

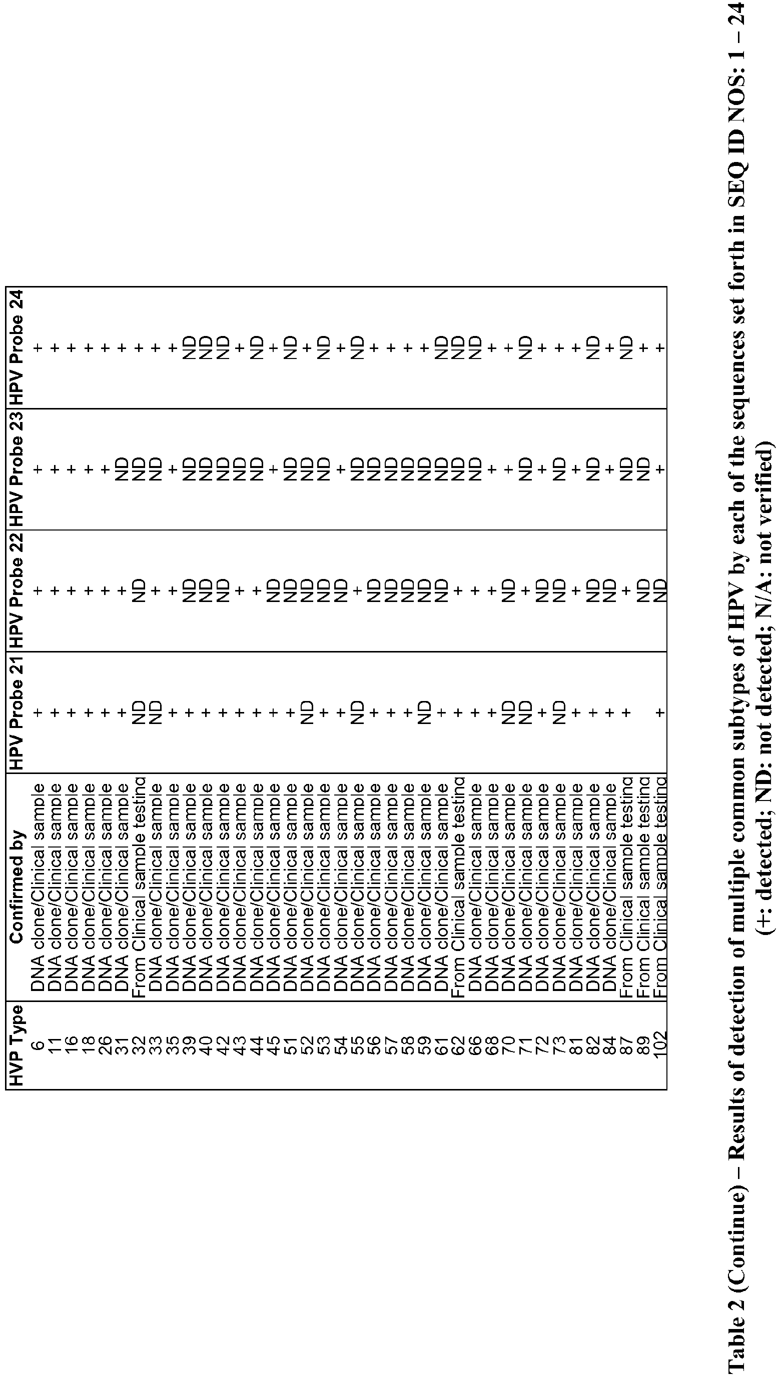

**Table 3 - Results of detection of multiple rare subtypes of HPV by each of the sequences set forth in SEQ ID NOS: 1 - 4 and 21 to 24 (D: deteced based on the alignment of the probe and HPV sequence which has <2bp mismatch; N/A: not verified)**

| **Extra HVP Type** | **Combined 4 probes (HPV 1-4)** | **HPV Probe 1** | **HPV Probe 2** | **HPV Probe 3** | **HPV Probe 4** | **Combined 4 probes (HPV 21-24)** | **HPV Probe 21** | **HPV Probe 22** | **HPV Probe 23** | **HPV Probe 24** |
|---|---|---|---|---|---|---|---|---|---|---|
| **1** | N/A | N/A | N/A | N/A | N/A | D | N/A | N/A | D | N/A |
| 2 | N/A | N/A | N/A | N/A | N/A | N/A | N/A | N/A | N/A | N/A |
| 3 | D | N/A | N/A | N/A | D | N/A | N/A | N/A | N/A | D |
| 5 | N/A | N/A | N/A | N/A | N/A | D | N/A | N/A | D | N/A |
| 7 | D | D | N/A | D | D | D | D | N/A | N/A | D |
| 8 | N/A | N/A | N/A | N/A | N/A | N/A | N/A | N/A | N/A | N/A |
| 9 | N/A | N/A | N/A | N/A | N/A | N/A | N/A | N/A | N/A | N/A |
| 10 | D | N/A | N/A | D | D | N/A | N/A | N/A | N/A | N/A |
| 12 | N/A | N/A | N/A | N/A | N/A | N/A | N/A | N/A | N/A | N/A |
| 13 | D | D | N/A | D | D | D | N/A | N/A | N/A | D |
| 14 | N/A | N/A | N/A | N/A | N/A | N/A | N/A | N/A | N/A | N/A |
| 17 | N/A | N/A | N/A | N/A | N/A | N/A | N/A | N/A | N/A | N/A |
| 19 | N/A | N/A | N/A | N/A | N/A | N/A | N/A | N/A | N/A | N/A |
| 20 | D | N/A | N/A | D | N/A | N/A | N/A | N/A | N/A | N/A |
| 21 | N/A | N/A | N/A | N/A | N/A | N/A | N/A | N/A | N/A | N/A |
| 22 | N/A | N/A | N/A | N/A | N/A | N/A | N/A | N/A | N/A | N/A |
| 23 | N/A | N/A | N/A | N/A | N/A | N/A | N/A | N/A | N/A | N/A |
| 24 | N/A | N/A | N/A | N/A | N/A | N/A | N/A | N/A | N/A | N/A |
| 25 | N/A | N/A | N/A | N/A | N/A | N/A | N/A | N/A | N/A | N/A |
| 27 | N/A | N/A | N/A | N/A | N/A | N/A | N/A | N/A | N/A | N/A |
| 28 | N/A | N/A | N/A | N/A | N/A | N/A | N/A | N/A | N/A | N/A |
| 29 | N/A | N/A | N/A | N/A | D | D | N/A | N/A | N/A | D |
| 30 | N/A | N/A | N/A | N/A | N/A | D | N/A | N/A | N/A | D |
| 32 | D | N/A | N/A | N/A | D | D | N/A | N/A | N/A | D |
| 34 | N/A | N/A | N/A | N/A | N/A | N/A | N/A | N/A | N/A | N/A |
| 36 | N/A | N/A | N/A | N/A | N/A | N/A | N/A | N/A | N/A | N/A |
| 37 | N/A | N/A | N/A | N/A | N/A | N/A | N/A | N/A | N/A | N/A |
| 38 | N/A | N/A | N/A | N/A | N/A | N/A | N/A | N/A | N/A | N/A |
| 41 | N/A | N/A | N/A | N/A | N/A | N/A | N/A | N/A | N/A | N/A |
| 47 | N/A | N/A | N/A | N/A | N/A | N/A | N/A | N/A | N/A | N/A |
| 48 | N/A | N/A | N/A | N/A | N/A | N/A | N/A | N/A | N/A | N/A |
| 49 | N/A | N/A | N/A | N/A | N/A | N/A | N/A | N/A | N/A | N/A |
| 50 | N/A | N/A | N/A | N/A | N/A | N/A | N/A | N/A | N/A | N/A |
| 57 | D | N/A | N/A | N/A | D | D | D | N/A | N/A | N/A |
| 64 | N/A | N/A | N/A | N/A | N/A | D | D | N/A | N/A | N/A |
| 67 | D | N/A | D | D | D | D | N/A | N/A | N/A | D |
| 83 | D | N/A | N/A | D | N/A | N/A | N/A | N/A | N/A | N/A |
| CP6108 | D | N/A | N/A | D | N/A | D | N/A | D | N/A | N/A |
| CP8061 | D | N/A | D | N/A | N/A | D | D | N/A | N/A | N/A |
| CP8304 | D | N/A | N/A | D | D | D | N/A | N/A | N/A | D |
| MM4 | D | N/A | D | N/A | N/A | N/A | N/A | N/A | N/A | N/A |
| MM7 | D | N/A | N/A | D | N/A | N/A | N/A | N/A | N/A | N/A |

**Table 4 - Detection results multiple subtypes of HPV by various combinations of 2 HPV probes**

| **HVP Type** | **Combined 4 probes (HPV 1+2)** | **HPV Probe 1** | **HPV Probe 2** | **Combined 4 probes (HPV 1+3)** | **HPV Probe 1** | **HPV Probe 3** | **Combined 2 probes (HPV 1 +4)** | **HPV Probe 1** | **HPV Probe 4** | **Combined 2 probes (HPV 1+16)** | **HPV Probe 1** | **HPV Probe 16** | **Combined 2 probes (HPV 1+24)** | **HPV Probe 1** | **HPV Probe 24** |
|---|---|---|---|---|---|---|---|---|---|---|---|---|---|---|---|
| **6** | + | + | + | + | + | + | + | + | + | + | + | + | + | + | + |
| **11** | + | + | + | + | + | + | + | + | + | + | + | ND | + | + | + |
| **16** | + | + | ND | + | + | + | + | + | + | + | + | ND | + | + | + |
| **18** | + | + | + | + | + | + | + | + | + | + | + | + | + | + | + |
| **26** | + | + | + | + | + | + | + | + | + | + | + | + | + | + | + |
| **31** | + | + | + | + | + | + | + | + | + | + | + | + | + | + | + |
| **32** | + | + | + | + | + | ND | + | + | ND | + | + | N/A | + | + | + |
| **33** | ND | ND | ND | + | ND | + | + | ND | + | + | ND | + | + | ND | + |
| **35** | + | + | + | + | + | + | + | + | + | + | + | + | + | + | + |
| **39** | + | + | + | + | + | ND | + | + | ND | + | + | + | + | + | ND |
| **40** | + | + | + | + | + | ND | + | + | + | + | + | ND | + | + | ND |
| **42** | + | + | + | + | + | + | + | + | ND | + | + | + | + | + | ND |
| **43** | + | + | + | + | + | + | + | + | + | + | + | ND | + | + | + |
| **44** | + | + | + | + | + | + | + | + | ND | + | + | + | + | + | ND |
| **45** | + | + | + | + | + | + | + | + | + | + | + | + | + | + | + |
| **51** | + | + | + | + | + | + | + | + | ND | + | + | ND | + | + | ND |
| **52** | + | + | + | + | + | ND | + | | + | + | + | + | + | + | + |
| **53** | + | + | ND | + | + | ND | + | + | ND | + | + | + | + | + | ND |
| **54** | + | + | + | + | + | ND | + | + | + | + | + | + | + | + | + |
| **55** | + | + | ND | + | + | + | + | + | ND | + | + | + | + | + | ND |
| **56** | + | + | ND | + | + | + | + | + | + | + | + | + | + | + | + |
| **57** | + | + | + | + | + | ND | + | + | + | + | + | ND | + | + | + |
| **58** | ND | ND | ND | + | ND | + | + | ND | + | + | ND | + | + | ND | + |
| **59** | + | + | + | + | + | ND | + | + | + | + | + | + | + | + | + |
| **61** | + | + | + | + | + | + | + | + | + | + | + | + | + | + | ND |
| **62** | + | + | ND | + | + | + | + | + | + | + | + | N/A | + | + | ND |
| **66** | + | + | ND | + | + | + | + | + | ND | + | + | ND | + | + | ND |
| **68** | + | + | + | + | + | ND | + | + | + | + | + | + | + | + | + |
| **70** | + | + | + | + | + | + | + | + | + | + | + | N/A | + | + | + |
| **71** | + | + | + | + | + | ND | + | + | ND | + | + | ND | + | + | ND |
| **72** | + | + | + | + | + | + | + | + | + | + | + | N/A | + | + | + |
| **73** | + | + | ND | + | + | + | + | + | + | + | + | ND | + | + | + |
| **81** | + | + | + | + | + | + | + | + | + | + | + | ND | + | + | + |
| **82** | + | + | + | + | + | ND | + | + | ND | + | + | ND | + | + | ND |
| **84** | + | ND | + | + | ND | + | ND | ND | ND | + | ND | + | + | ND | + |
| **87** | + | + | ND | + | + | + | + | + | + | + | + | N/A | + | + | ND |
| **89** | + | + | + | + | + | ND | + | + | + | + | + | N/A | + | + | + |
| **102** | + | + | + | + | + | ND | + | + | + | + | + | N/A | + | + | + |
| **Total subtype** | **36** | **35** | **28** | **38** | **35** | **25** | **37** | **35** | **27** | **38** | **35** | **20** | **38** | **35** | **25** |

**Table 5 - Detection results multiple subtypes of HPV by various combinations of 3 HPV probes**

| **HVP Type** | **Combined 3 probes (HPV 2+3+5)** | **HPV Probe 2** | **HPV Probe 3** | **HPV Probe 5** | **Combined 3 probes (HPV 6+20+21)** | **HPV Probe 6** | **HPV Probe 20** | **HPV Probe 21** |
|---|---|---|---|---|---|---|---|---|
| **6** | + | + | + | + | + | + | + | + |
| **11** | + | + | + | + | + | + | ND | + |
| **16** | + | ND | + | + | + | + | ND | + |
| **18** | + | + | + | + | + | + | + | + |
| **26** | + | + | + | + | + | + | + | + |
| **31** | + | + | + | + | + | + | + | + |
| **32** | + | + | ND | N/A | N/A or ND | N/A | N/A | ND |
| **33** | + | ND | + | + | + | ND | + | ND |
| **35** | + | + | + | + | + | ND | + | + |
| **39** | + | + | ND | + | + | + | + | + |
| **40** | + | + | ND | + | + | ND | ND | + |
| **42** | + | + | + | ND | + | + | + | + |
| **43** | + | + | + | + | + | + | ND | + |
| **44** | + | + | + | ND | + | + | + | + |
| **45** | + | + | + | + | + | ND | + | + |
| **51** | + | + | + | ND | + | + | ND | + |
| **52** | + | + | ND | + | + | + | ND | ND |
| **53** | + | ND | ND | + | + | + | ND | + |
| **54** | + | + | ND | + | + | ND | N/A | + |
| **55** | + | ND | + | ND | + | + | N/A | ND |
| **56** | + | ND | + | + | + | + | ND | + |
| **57** | + | + | ND | + | + | ND | ND | + |
| **58** | + | ND | + | + | + | ND | ND | + |
| **59** | + | + | ND | ND | + | ND | + | ND |
| **61** | + | + | + | + | + | + | N/A | + |
| **62** | + | ND | + | + | + | + | N/A | + |
| **66** | + | ND | + | + | + | + | ND | + |
| **68** | + | + | ND | + | + | + | ND | + |
| **70** | + | + | + | ND | + | + | N/A | ND |
| **71** | + | + | ND | ND | + | ND | + | ND |
| **72** | + | + | + | + | + | + | N/A | + |
| **73** | + | ND | + | ND | + | + | + | ND |
| **81** | + | + | + | + | + | + | ND | + |
| **82** | + | + | ND | ND | + | + | ND | + |
| **84** | + | + | + | ND | + | ND | ND | + |
| **87** | + | ND | + | N/A | + | N/A | N/A | + |
| **89** | + | + | ND | N/A | + | N/A | N/A | + |
| **102** | + | + | ND | N/A | + | N/A | N/A | + |
| **Total subtype** | **38** | **28** | **25** | **24** | **37** | **24** | **13** | **30** |

**Table 6 - Detection results multiple subtypes of HPV by various combinations of 4 HPV probes**

| **HVP Type** | **Combined 4 probes (HPV 2+6+22+24)** | **HPV Probe 2** | **HPV Probe 6** | **HPV Probe 22** | **HPV Probe 24** | **Combined 4 probes (HPV 16+17+21+24)** | **HPV Probe 16** | **HPV Probe 17** | **HPV Probe 21** | **HPV Probe 24** |
|---|---|---|---|---|---|---|---|---|---|---|
| **6** | + | + | + | + | + | + | + | ND | + | + |
| **11** | + | + | + | + | + | + | ND | ND | + | + |
| **16** | + | ND | + | + | + | + | ND | ND | + | + |
| **18** | + | + | + | + | + | + | + | + | + | + |
| **26** | + | + | + | + | + | + | + | ND | + | + |
| **31** | + | + | + | + | + | + | + | + | + | + |
| **32** | + | + | N/A | ND | + | + | N/A | N/A | ND | + |
| **33** | + | ND | ND | + | + | + | + | + | ND | + |
| **35** | + | + | ND | + | + | + | + | ND | + | + |
| **39** | + | + | + | ND | ND | + | + | + | + | ND |
| **40** | + | + | ND | ND | ND | + | ND | ND | + | ND |
| **42** | + | + | + | ND | ND | + | + | + | + | ND |
| **43** | + | + | + | + | + | + | ND | ND | + | + |
| **44** | + | + | + | + | ND | + | + | ND | + | ND |
| **45** | + | + | ND | ND | + | + | + | ND | + | + |
| **51** | + | + | + | ND | ND | + | ND | ND | + | ND |
| **52** | + | + | + | ND | + | + | + | + | ND | + |
| **53** | + | ND | + | ND | ND | + | + | + | + | ND |
| **54** | + | + | ND | ND | + | + | + | N/A | + | + |
| **55** | + | ND | + | + | ND | + | + | + | ND | ND |
| **56** | + | ND | + | ND | + | + | + | ND | + | + |
| **57** | + | + | ND | ND | + | + | ND | + | + | + |
| **58** | + | ND | ND | ND | + | + | + | ND | + | + |
| **59** | + | + | ND | ND | + | + | + | ND | ND | + |
| **61** | + | + | + | ND | ND | + | + | N/A | + | ND |
| **62** | + | ND | + | + | ND | + | N/A | N/A | + | ND |
| **66** | + | ND | + | + | ND | + | ND | ND | + | ND |
| **68** | + | + | + | + | + | + | + | ND | + | + |
| **70** | + | + | + | ND | + | + | N/A | + | ND | + |
| **71** | + | + | ND | + | ND | + | ND | + | ND | ND |
| **72** | + | + | + | ND | + | + | N/A | N/A | + | + |
| **73** | + | ND | + | ND | + | + | ND | + | ND | + |
| **81** | + | + | + | + | + | + | ND | + | + | + |
| **82** | + | + | + | ND | ND | + | ND | + | + | ND |
| **84** | + | + | ND | ND | + | + | + | ND | + | + |
| **87** | + | ND | N/A | + | ND | + | N/A | N/A | + | ND |
| **89** | + | + | N/A | ND | + | + | N/A | N/A | + | + |
| **102** | + | + | N/A | ND | + | + | N/A | N/A | + | + |
| **Total subtype** | **38** | **28** | **24** | **17** | **25** | **38** | **20** | **14** | **30** | **25** |

The results of the detection of multiple subtypes of HPV by each of the SEQ ID NOS: 1-24 are shown in **Tables 2** and **3,** in which **Table 2** shows the results for the detection of common 38 subtypes of HPV, while **Table 3** shows the results for rare subtypes of HPV. The results show that each of the sequences set forth in SEQ ID NOS: 1 - 24 is able to detect multiple subtypes of HPV. Further, various combinations of at least 2 to 4 of the sequences set forth in SEQ ID NOS: 1 - 24 is able to detect at least 36 common subtypes of HPV, which can be noted from the results of **Tables 2** and **3.** For example and as shown in **Table 4,** a combination of HPV probes 1 and 2 can detect at least 36 subtypes of HPV, while other combinations of 2 HPV probes of the current disclosure can detect at least 37 or 38 subtypes of HPV. **Table 5** shows similar results for various combinations of 3 HPV probes of the current disclosure, that is, a combination of at least 3 HPV probes to detect at least 36, more specifically 37 or 38, subtypes of HPV. Particularly, the use of a combination of 4 HPV probes having respective sequences set forth in SEQ ID NOS: 1 - 4, that is, one probe has a sequence sets forth in SEQ ID NO 1, one probe has a sequence sets forth in SEQ ID NO 2, one probe has a sequence sets forth in SEQ ID NO 3, and one probe has a sequence sets forth in SEQ ID NO 4, is able to detect 38 common subtypes of HPV, including the high risk types 16, 18, 31, 33, 35, 45, 51, 52, 58, and 59. However, other than the various combinations shown in **Table 6,** it can be noted from the results of **Table 2** that other combinations of four HPV probes are also capable of detecting at least 36, more specifically 38, subtypes of HPV, for example, SEQ ID NOS: 1 - 3 and 5; SEQ ID NOS: 1, 2, 4, and 5; SEQ ID NOS: 1 - 3 and 6; SEQ ID NOS: 1, 2, 4, and 7.

As known in the art, sequences complementary to any of the sequences set forth in SEQ ID NOS: 1 - 24 are also useful for either detecting HPV, or for making nucleic acid molecules having the sequences set forth in SEQ ID NOS: 1 - 24.

If desired, RNA of having the sequences set forth in SEQ ID NOS: 1 - 24 may be used, although DNA is generally preferred due to higher stability in the resulting detection kit and chip. However, it is understood by an ordinary skilled in the art that the term DNA wherein the nucleotide base includes those modified by various groups used for labeling signal generating; those used for modulating the Tm value of the annealing temperature and/or increase the stability of the molecules such as Locked Nucleic Acid (LNA) and Peptide Nucleic Acid (PNA) and RNA molecule.

The detection kit of the current disclosure includes: a DNA chip with at least one probe that have nucleotide sequences complementary to DNA of HPV, more specifically the at least one HPV probe being an isolated nucleic acid molecule selected from the group consisting of: (a) a sequence selected from the group consisting of SEQ ID NOS: 1-24; and (b) a sequence fully complementary to any of said nucleic acid molecule of (a). Primers for amplifying DNA obtained from clinical samples by PCR are also included. In a particular example of the current disclosure, known primers for amplifying DNA from the samples for detecting HPV shown in **Table 7** below are used:

**Table 7 - sequences of nucleotides for amplification of HPV (M=A/C; W=A/T; Y=T+C)**

| **Name** | **SEQ ID NO** | **Sequence** | **5' Modification** | **Used as** | **Length** |
|---|---|---|---|---|---|
| HPVF | 25 | GCMCA GGGWC ATAAY AATGG | Biotin | Primer | 20 |
| HPVR | 26 | CGTCC MARRG GAWAC TGATC | Biotin | Primer | 20 |
| HPVR2 | 27 | GCGAC CCAAT GCAAA TTGGT | N/A | Primer | 20 |
| HPVP | 28 | GGTCC MARRG GAWAC TGATC | Biotin | Primer | 20 |

The detection kit also includes labeling agents for labeling amplified DNA hybridized with the probes of the DNA chip. As known in the field, the DNA chip may further include position markers to locate probes, and staining or labeling is performed by using labeling agents such as biotin-binding material, for example a conjugate of a fluorophore and a protein with biotin-binding sites such as streptavidin-R-phycoerythrin, horseradish peroxidase, digoxigenin, chemiluminescence or fluorescent (FAM, HEX, Cys3, Cys5) and detected with a scanner..

Additionally and as known in the art, globin primers and probe may be included for monitoring the quality of the PCR process. These globin primers and probe are used to check whether the sample has DNA, and whether the DNA in the sample can be satisfactorily amplified. Examples of globin primers and probe are listed in **Table 8** below, which can be used in the current disclosure:

**Table 8 - sequences of globin primers and probe**

| **Name** | **SEQ ID NO** | **Sequence** | **5' Modification** | **Used as** | **Length** |
|---|---|---|---|---|---|
| IC-R | 29 | CCTTG ATACC AACCT GCCCA G | Biotin | Primer | 21 |
| IC-F | 30 | CCTTG ATACC AACCT GCCCA G | None | Primer | 21 |
| IC-Probe | 31 | AAGGT GAACG TGGAT GAAGT TGGTG G | Amine | Probe | 26 |
| IC-F (New) | 32 | GAAGA GCCAA GGACA GGTAC | None | Primer | 20 |

The forward primer IC-F (New) is designed in the current disclosure that can replace the existing forward primer IC-F. With the use of this new primer IC-F (New), a larger PCR amplification is resulted, and is more compatible to the HPV detection kit of the current disclosure and hence to improve the kit detection efficiency.

The DNA chip used in the detection kit of the current invention may be prepared with the following known processes:
1. 5' terminal amine-linked DNA probes which have an isolated nucleic acid molecule selected from the group consisting of: (a) a sequence selected from the group consisting of SEQ ID NOS: 1-24; and (b) a sequence fully complementary to any of said nucleic acid molecule of (a) are prepared.
2. The prepared DNA probes are then affixed to an aldehyde-derivatized solid surface, for example glass. The probes can be affixed to the surface of the solid support via Schiffs base reaction between an aldehyde group on the surface of solid support and an amine group at 5' terminal of the probes under 20 to 35°C and 50 to 70% humidity, while controlling the concentration of probes, for example in a range of preferably 100 to 300 µmol/µL, more preferably 200 µmol/µL.
3. Excessive aldehydes not reacted with amine on the surface of the solid support are reduced by a suitable reducing agent, for example NaBH (sodium borohydride), finally to prepare the DNA chip.

HPV infection can be detected by the HPV detection kit of the current disclosure First, DNA in a sample, for example a clinical sample, is amplified by PCR with suitable primers for example those in **Table 2.** The amplified DNA are then applied to the DNA chip for hybridization. Finally, the amplified DNA are bound on the surface of the DNA chip after labeling. For example, the amplified sample DNA hybridized with the probes can be labeled with, for example, Streptavidin-R-phycoerythrin and detected with a laser scanner.

Other than the above, the sequences of the HPV probes can be used in the reversed flow-through detection methods developed by the inventors and described in WO 2007/115491A and WO 2009/044370A, which allow 64 samples to be detected simultaneously, and results of the detection can be available within 5 to 15 minutes.

As shown above, the current invention provides various sequences capable of detecting HPV, in which each of the sequences is able to detect multiple subtypes of HPV. With the sequences of the current invention, it is possible to detect commonly seen 38 subtypes of HPV, including those of high risk, with various combinations of four sequences of the current invention. This allows reduction of the costs of the resulting detection kits and DNA chips. Further, as relatively few sequences are used as detection probe, complexity of the detection process can be reduced, and more accurate results can be obtained. These can reduce the overall costs of HPV detection, which is important for developing countries. After HPV infection is detected in a patient, the specific subtype of the HPV infection in the patient can then be detected by genotyping if necessary.

While the preferred embodiment of the present invention has been described in detail by the examples, it is apparent that modifications and adaptations of the present invention will occur to those skilled in the art. Furthermore, the embodiments of the present invention shall not be interpreted to be restricted by the examples or figures only. The scope of the present invention is as set forth in the following claims.

## Claims

1. An isolated nucleic acid molecule consisting of a sequence selected from the group consisting of SEQ ID NOS: 1, 2 and 4 as shown in table 1, wherein a + sign preceding a base of SEQ ID NOS: 1, 2 and 4 means that the base is an LNA modified base.

2. The isolated nucleic acid molecule of claim 1, wherein the sequence is an HPV probe sequence consisting of SEQ ID NO: 1 as shown in table 1.

3. The isolated nucleic acid molecule of claim 1, wherein the sequence is an HPV probe sequence consisting of SEQ ID NO: 2 as shown in table 1.

4. The isolated nucleic acid molecule of claim 1, wherein the sequence is an HPV probe sequence consisting of SEQ ID NO: 4 as shown in table 1.

5. An HPV detection kit including:
at least one HPV probe for detecting HPV, each HPV probe being an isolated nucleic acid molecule consisting of a sequence selected from the group consisting of SEQ ID NOS: 1, 2 and 4 as shown in table 1; wherein a + sign preceding a base of SEQ ID NOS: 1, 2 and 4 means that the base is an LNA modified base.
primers for amplifying HPV in sample DNA by polymerase chain reaction; and
labels for labeling DNA that is amplified and hybridized to a DNA chip.

6. A DNA chip for detecting at least two subtypes of HPV including at least one HPV probe, each HPV probe being an isolated nucleic acid molecule consisting of a sequence selected from the group consisting of SEQ ID NOS: 1, 2 and 4 as shown in table 1; wherein a + sign preceding a base of SEQ ID NOS: 1, 2 and 4 means that the base is an LNA modified base.

7. A method of detecting presence of HPV in a sample, including the steps of:
amplifying DNA of HPV present in the sample by means of a polymerase chain reaction;
detecting HPV with at least one HPV probe, each HPV probe being an isolated nucleic acid molecule consisting of a sequence selected from the group consisting of SEQ ID NOS: 1, 2 and 4 as shown in table 1; wherein a + sign preceding a base of SEQ ID NOS: 1, 2 and 4 means that the base is an LNA modified base.

8. The HPV detection kit of claim 5, DNA chip of claim 6 or method of claim 7, wherein the detection kit, the DNA chip and he method includes a combination of four HPV probes to detect HPV, said four HPV probes being sequences consisting of SEQ ID NOS. 1-3 and 5, as shown in table 1; sequences consisting of SEQ ID NOS. 1-3 and 6, as shown in table 1; sequences consisting of SEQ IS NOS. 1, 2, 4 and 5, as shown in table 1; or sequences consisting of SEQ ID NOS : 1, 2, 4 and 7, as shown in table 1.

9. The HPV detection kit of claim 5, DNA chip of claim 6 or method of claim 7, wherein the detection kit, the DNA chip and the method includes a combination of four HPV probes to detect HPV, said four HPV probes consisting of sequences consisting of SEQ ID NOS: 1-4, as shown in table 1, or sequences consisting of SEQ ID NOS: 2, 6, 22 and 24, as shown in table 1.

10. The HPV detection kit of claim 5, DNA chip of claim 6 or method of claim 7, wherein the detection kit, the DNA chip and the method includes a combination of three HPV probes to detect HPV, the combination of the said three HPV probes consists of SEQ ID NOS: 2, 3 and 5, as shown in table 1.

11. The HPV detection kit of claim 5, DNA chip of claim 6 or method of claim 7, wherein the detection kit, the DNA chip and the method includes a combination of two HPV probes to detect HPV, the combination of said two HPV probes consisting of SEQ ID NO: 1 and one of SEQ ID NOS: 2, 3, 4, 16 and 24, as shown in table 1.

12. The HPV detection kit, DNA chip or method of any one of the claims 7 to 11, wherein the isolated nucleic acid molecule is DNA or RNA.

13. An HPV detection kit including:
at least two HPV probes for detecting HPV, one of said at least two HPV probes being an isolated nucleic acid molecule consisting of SEQ ID NO: 1, as shown in table 1, the other one of said at least two HPV probes being an isolated nucleic acid molecule consisting of SEQ ID NOS: 2, 3, 4, 16, or 24, as shown in table 1, wherein a + sign preceding a base of SEQ ID NOS: 1, 2, 3, 4, 16 and 24 means that the base is an LNA modified base;
primers for amplifying HPV in sample DNA by polymerase chain reaction; and
labels for labeling DNA that is amplified and hybridized to a DNA chip.

14. A method of detecting presence of HPV, including the steps of:
amplifying DNA of HPV present in the sample by means of a polymerase chain reaction;
detecting HPV with at least two HPV probes, one of the said HPV probes being an isolated nucleic acid molecule consisting of SEQ ID NO:1 as shown in table 1, the other one of said at least two HPV probes being an isolated nucleic acid molecule consisting of SEQ NOS: 2, 3, 4, 16, or 24, as shown in table 1 when said one of said at least two HPV probes is SEQ ID NO: 1, as shown in table 1; wherein a + sign preceding a base of SEQ ID NOS: 1, 2, 3, 4, 16 and 24 means that the base is an LNA modified base.

## Patentansprüche

1. Isoliertes Nukleinsäuremolekül, bestehend aus einer Sequenz, ausgewählt aus der Gruppe, bestehend aus SEQ ID-NR.: 1, 2 und 4, wie in Tabelle 1 gezeigt, wobei ein +-Zeichen vor einer Base von SEQ ID-NR.: 1, 2 und 4 bedeutet, dass die Base eine LNA-modifizierte Base ist.

2. Isoliertes Nukleinsäuremolekül nach Anspruch 1, wobei die Sequenz eine HPV-Sondensequenz ist, bestehend aus SEQ ID-NR.: 1, wie in Tabelle 1 gezeigt.

3. Isoliertes Nukleinsäuremolekül nach Anspruch 1, wobei die Sequenz eine HPV-Sondensequenz ist, bestehend aus SEQ ID-NR.: 2, wie in Tabelle 1 gezeigt.

4. Isoliertes Nukleinsäuremolekül nach Anspruch 1, wobei die Sequenz eine HPV-Sondensequenz ist, bestehend aus SEQ ID-NR.: 4, wie in Tabelle 1 gezeigt.

5. HPV-Nachweiskit, einschließend:
mindestens eine HPV-Sonde zum Nachweisen von HPV, wobei jede HPV-Sonde ein isoliertes Nukleinsäuremolekül ist, bestehend aus einer Sequenz, ausgewählt aus der Gruppe, bestehend aus SEQ ID-NR.: 1, 2 und 4, wie in Tabelle 1 gezeigt; wobei ein +-Zeichen vor einer Base von SEQ ID-NR.: 1, 2 und 4 bedeutet, dass die Base eine LNA-modifizierte Base ist.
Primer zum Amplifizieren von HPV in Probe-DNA durch Polymerase-Kettenreaktion und
Markierungen zum Markieren von DNA, die amplifiziert und zu einem DNA-Chip hybridisiert wird.

6. DNA-Chip zum Nachweisen von mindestens zwei Unterarten von HPV, einschließlich mindestens einer HPV-Sonde, wobei jede HPV-Sonde ein isoliertes Nukleinsäuremolekül ist, bestehend aus einer Sequenz, ausgewählt aus der Gruppe, bestehend aus SEQ ID-Nr.: 1, 2 und 4, wie in Tabelle 1 gezeigt; wobei ein +-Zeichen vor einer Base von SEQ ID-NR.: 1, 2 und 4 bedeutet, dass die Base eine LNA-modifizierte Base ist.

7. Verfahren zum Erkennen des Vorliegens von HPV in einer Probe, einschließlich der folgenden Schritte:
Amplifizieren von DNA von HPV, die in der Probe vorliegt, mittels einer Polymerase-Kettenreaktion;
Erkennen von HPV mit mindestens einer HPV-Sonde, wobei jede HPV-Sonde ein isoliertes Nukleinsäuremolekül ist, bestehend aus einer Sequenz, ausgewählt aus der Gruppe, bestehend aus SEQ ID-NR.: 1, 2 und 4, wie in Tabelle 1 gezeigt; wobei ein +-Zeichen vor einer Base von SEQ ID-NR.: 1, 2 und 4 bedeutet, dass die Base eine LNA-modifizierte Base ist.

8. HPV-Nachweiskit nach Anspruch 5, DNA-Chip nach Anspruch 6 oder Verfahren nach Anspruch 7, wobei der Nachweiskit, der DNA-Chip und das Verfahren eine Kombination aus vier HPV-Sonden einschließt, um HPV zu erkennen, wobei die vier HPV-Sonden Sequenzen sind, bestehend aus SEQ ID-NR.: 1-3 und 5, wie in Tabelle 1 gezeigt; Sequenzen, bestehend aus SEQ ID-NR. 1-3 und 6, wie in Tabelle 1 gezeigt; Sequenzen, bestehend aus SEQ ID-NR.: 1, 2, 4 und 5, wie in Tabelle 1 gezeigt, oder Sequenzen, bestehend aus SEQ ID-NR.: 1, 2, 4 und 7, wie in Tabelle 1 gezeigt.

9. HPV-Nachweiskit nach Anspruch 5, DNA-Chip nach Anspruch 6 oder Verfahren nach Anspruch 7, wobei der Nachweiskit, der DNA-Chip und das Verfahren eine Kombination aus vier HPV-Sonden einschließt, um HPV zu erkennen, wobei die vier HPV-Sonden aus Sequenzen bestehen, bestehend aus SEQ ID-NR.: 1-4, wie in Tabelle 1 gezeigt, oder Sequenzen, bestehend aus SEQ ID-NR.: 2, 6, 22 und 24, wie in Tabelle 1 gezeigt.

10. HPV-Nachweiskit nach Anspruch 5, DNA-Chip nach Anspruch 6 oder Verfahren nach Anspruch 7, wobei der Nachweiskit, der DNA-Chip und das Verfahren eine Kombination aus drei HPV-Sonden einschließen, um HPV zu erkennen, wobei die Kombination der drei HPV-Sonden aus SEQ ID NR.: 2, 3 und 5 besteht, wie in Tabelle 1 gezeigt.

11. HPV-Nachweiskit nach Anspruch 5, DNA-Chip nach Anspruch 6 oder Verfahren nach Anspruch 7, wobei der Nachweiskit, der DNA-Chip und das Verfahren eine Kombination aus zwei HPV-Sonden einschließt, um HPV zu erkennen, wobei die Kombination der zwei HPV-Sonden aus SEQ ID-NR.: 1 und eine aus SEQ ID-NR.: 2, 3, 4, 16 und 24 besteht, wie in Tabelle 1 gezeigt.

12. HPV-Nachweiskit, DNA-Chip oder Verfahren nach einem der Ansprüche 7 bis 11, wobei das isolierte Nukleinsäuremolekül DNA oder RNA ist.

13. HPV-Nachweiskit, einschließend:
mindestens zwei HPV-Sonden zum Erkennen von HPV, wobei eine der mindestens zwei HPV-Sonden ein isoliertes Nukleinsäuremolekül ist, bestehend aus SEQ ID-NR.: 1, wie in Tabelle 1 gezeigt, wobei die andere der mindestens zwei HPV-Sonden ein isoliertes Nukleinsäuremolekül ist, das aus SEQ ID-NR.: 2, 3, 4, 16 oder 24 besteht, wie in Tabelle 1 gezeigt, wobei ein +-Zeichen vor einer Base der SEQ ID-NR.: 1, 2, 3, 4, 16 und 24 bedeutet, dass die Base eine LNA-modifizierte Base ist;
Primer zum Amplifizieren von HPV in Probe-DNA durch Polymerase-Kettenreaktion und
Markierungen zum Markieren von DNA, die amplifiziert und zu einem DNA-Chip hybridisiert wird.

14. Verfahren zum Erkennen des Vorliegens von HPV, einschließlich der folgenden Schritte:
Amplifizieren von DNA von HPV, das in der Probe vorliegt, mittels einer Polymerase-Kettenreaktion;
Erkennen von HPV mit mindestens zwei HPV-Sonden, wobei eine der HPV-Sonden ein isoliertes Nukleinsäuremolekül ist, bestehend aus SEQ ID-NR.: 1, wie in Tabelle 1 gezeigt, wobei die andere der mindestens zwei HPV-Sonden ein isoliertes Nukleinsäuremolekül ist, bestehend aus SEQ ID-NR.: 2, 3, 4, 16 oder 24, wie in Tabelle 1 gezeigt, wenn die eine der mindestens zwei HPV-Sonden SEQ ID-NR.: 1 ist, wie in Tabelle 1 gezeigt; wobei ein +-Zeichen vor einer Base der SEQ ID-NR.: 1, 2, 3, 4, 16 und 24 bedeutet, dass die Base eine LNA-modifizierte Base ist.

## Revendications

1. Molécule d'acide nucléique isolée comprenant une séquence sélectionnée à partir du groupe constitué par les SEQ ID NO 1, 2 et 4 comme indiqué dans le tableau 1, dans lequel un signe + précédant une base des SEQ ID NO 1, 2 et 4 signifie que la base est une base modifiée LNA.

2. Molécule d'acide nucléique isolée de la revendication 1 dans laquelle la séquence est une séquence de sondes HPV constituée par la SEQ ID NO 1 comme indiqué dans le tableau 1.

3. Molécule d'acide nucléique isolée de la revendication 1 dans laquelle la séquence est une séquence de sondes HPV constituée par la SEQ ID NO 2 comme indiqué dans le tableau 1.

4. Molécule d'acide nucléique isolée de la revendication 1 dans laquelle la séquence est une séquence de sondes HPV constituée par la SEQ ID NO 4 comme indiqué dans le tableau 1.

5. Kit de détection d'HPV incluant :
au moins une sonde HPV pour la détection d'HPV, chaque sonde HPV étant une molécule d'acide nucléique isolée comprenant une séquence sélectionnée à partir du groupe constitué par les SEQ ID NO 1, 2 et 4 comme indiqué dans le tableau 1 ; dans lequel un signe + précédant une base des SEQ ID NO 1, 2 et 4 signifie que la base est une base modifiée LNA ;
des amorces pour l'amplification d'HPV dans un échantillon d'ADN par réaction en chaîne de la polymérase ; et
des étiquettes pour le marquage de l'ADN qui est amplifié et hybridé en puce à ADN.

6. Puce à ADN pour la détection d'au moins deux sous-types d'HPV comprenant au moins une sonde HPV, chaque sonde HPV étant une molécule d'acide nucléique isolée comprenant une séquence sélectionnée à partir du groupe constitué par les SEQ ID NO 1, 2 et 4 comme indiqué dans le tableau 1 ; dans lequel un signe + précédant une base des SEQ ID NO 1, 2 et 4 signifie que la base est une base modifiée LNA.

7. Procédé de détection de la présence d'HPV dans un échantillon, incluant les étapes consistant à :
amplifier l'ADN de l'HPV présent dans l'échantillon par une réaction en chaîne de la polymérase ;
détecter l'HPV avec au moins une sonde HPV, chaque sonde HPV étant une molécule d'acide nucléique isolée comprenant une séquence sélectionnée à partir du groupe constitué par les SEQ ID NO 1, 2 et 4 comme indiqué dans le tableau 1 ; dans lequel un signe + précédant une base des SEQ ID NO 1, 2 et 4 signifie que la base est une base modifiée LNA.

8. Kit de détection d'HPV de la revendication 5, puce à ADN de la revendication 6 ou procédé de la revendication 7 dans lequel le kit de détection, la puce à ADN et le procédé incluent une combinaison de quatre sondes pour la détection de l'HPV, lesdites quatre sondes HPV étant des séquences constituées des SEQ ID NO 1-3 et 5, comme indiqué dans le tableau 1 ; des séquences constituées des SEQ ID NO 1-3 et 6, comme indiqué dans le tableau 1 ; des séquences constituées des SEQ ID NO 1, 2, 4 et 5, comme indiqué dans la tableau 1 ; ou des séquences constituées des SEQ ID NO 1, 2, 4 et 7, comme indiqué dans le tableau 1.

9. Kit de détection d'HPV de la revendication 5, puce à ADN de la revendication 6 ou procédé de la revendication 7 dans lequel le kit de détection, la puce à ADN et le procédé incluent une combinaison de quatre sondes pour la détection de l'HPV, lesdites quatre sondes HPV étant des séquences constituées des SEQ ID NO 1-4, comme indiqué dans le tableau 1, ou des séquences constituées des SEQ ID NO 2, 6, 22 et 24, comme indiqué dans le tableau 1.

10. Kit de détection d'HPV de la revendication 5, puce à ADN de la revendication 6 ou procédé de la revendication 7 dans lequel le kit de détection, la puce à ADN et le procédé incluent une combinaison de trois sondes HPV pour la détection de l'HPV, la combinaison desdites trois sondes HPV étant constituée des SEQ ID NO 2, 3 et 5, comme indiqué dans le tableau 1.

11. Kit de détection d'HPV de la revendication 5, puce à ADN de la revendication 6 ou procédé de la revendication 7 dans lequel le kit de détection, la puce à ADN et le procédé incluent une combinaison de deux sondes HPV pour la détection de l'HPV, la combinaison desdites deux sondes HPV étant constituée de la SEQ ID NO 1 et une des SEQ ID NO 2, 3, 4, 16 et 24, comme indiqué dans le tableau 1.

12. Kit de détection d'HPV, puce à ADN ou procédé de l'une quelconque des revendications 7 à 11 dans lequel la molécule d'acide nucléique isolée est de l'ADN ou de l'ARN.

13. Kit de détection d'HPV incluant :
au moins deux sondes HPV pour la détection de l'HPV, une desdites au moins deux sondes HPV étant une molécule d'acide nucléique isolée de la SEQ ID NO 1, comme indiqué dans le tableau 1, l'autre desdites au moins deux sondes HPV étant une molécule d'acide nucléique isolée constituée des SEQ ID NO 2, 3, 4, 16 ou 24, comme indiqué dans le tableau 1, dans lequel un signe + précédant une base des SEQ ID NO 1, 2, 3, 4, 16 et 24 signifie que la base est une base modifiée LNA ;
des amorces pour l'amplification d'HPV dans un échantillon d'ADN par réaction en chaîne de la polymérase ; et
des étiquettes pour le marquage de l'ADN qui est amplifié et hybridé en puce à ADN.

14. Procédé de détection de la présence d'HPV comprenant les étapes consistant à :
amplifier l'ADN de l'HPV présent dans l'échantillon par une réaction en chaîne de la polymérase ;
détecter l'HPV avec au moins une des deux sondes HPV, une desdites sondes HPV étant une molécule d'acide nucléique isolée étant constituée de la SEQ ID NO 1 comme indiqué dans le tableau 1, l'autre desdites au moins deux sondes HPV étant une molécule d'acide nucléique isolée étant constituée des SEQ ID NO 2, 3, 4, 16 ou 24 comme indiqué dans le tableau 1 lorsqu'une desdites au moins au moins deux sondes HPV est la SEQ ID NO 1, comme indiqué dans le tableau 1 ; dans lequel un signe + précédant une base des SEQ ID NO 1, 2, 3, 4, 16 et 24 signifie que la base est une base modifiée LNA.
